# EUROPEAN PATENT APPLICATION

(11) **EP 4 375 674 A1**
(43) Date of publication of application: **29.05.2024**
(21) Application number: 22845699.2
(22) Date of filing: 01.06.2022
(51) Int. Cl.: G01N 35/00, G16H 10/40

(54) **AUTOMATIC ANALYSIS DEVICE AND SAMPLE INFORMATION DISPLAY METHOD**

(30) Priority: 20.07.2021 JP 2021119198
(71) Applicant: HITACHI HIGH-TECH CORPORATION, Tokyo 105-6409 (JP)
(72) Inventor: ITOU Kenta, Tokyo 105-6409 (JP)
(74) Representative: Strehl Schübel-Hopf & Partner
(86) International application number: PCT/JP2022/022318
(87) International publication number: WO 2023/002762

(57) **Abstract**

Provided is an automatic analyzer having an information leakage prevention function for managing viewing of specimen information by a user. For this purpose, when a logged-in user causes a display device to display specimen information on a predetermined specimen ID, a computer refers to reference authority information set for each analysis item regarding a user ID of the user and reference classification information in specimen ID data on the predetermined specimen ID, and determines whether a measurement value of an analysis item can be displayed. For example, a measurement value of an analysis item requested for the predetermined specimen ID is hidden on the display device when reference classification information on the analysis item is a classification indicating presence of a reference restriction in the specimen ID data and reference authority information set for the analysis item regarding the user ID is a classification indicating absence of a reference authority.

## Description

### Technical Field

The present invention relates to an automatic analyzer that performs quantitative and qualitative analysis of a specimen (biological sample) such as blood or urine, and a specimen information display method for displaying specimen information including a measurement result of an automatic analyzer.

### Background Art

An automatic analyzer measures a specimen of a patient and outputs an obtained analysis result along with information on the specimen to a display screen. These pieces of information include personal information for identifying the patient who is a provider of the specimen, and thus should be handled with great care. In addition, the analysis result obtained by the automatic analyzer may contain information that directly indicates a health status of the patient, such as presence or absence of a tumor or presence or absence of an infectious disease, and therefore should be handled with care.

PTL 1 discloses an automatic analyzer configured to perform authentication using a user ID and a password and change setting of whether to output only an inspection result or whether to output patient attribute information including an ID, name, and the like of a patient along with the inspection result depending on whether a type of a logged-in user is a general user such as a laboratory technician or a technician, so as to prevent leakage of personal information.

### Citation List

### Patent Literature

PTL 1: JP2011-33536A

### Summary of Invention

### Technical Problem

In recent years, stringency of information management tends to be strictly required from a viewpoint of information security. On the other hand, due to the spread of diverse work styles, even in a laboratory where specimen analysis is performed, it is expected that it is common for various employment types of laboratory technicians and others to come in and out of the laboratory. Currently, patient information and analysis result information are prevented from leaking outside the laboratory by a confidentiality agreement signed with the laboratory technician, but it is considered that measures against information leakage will be required as a function of an automatic analyzer in the future.

In PTL 1, whether or not to output the patient attribute information is controlled based on the user ID and password. However, in this method, display is controlled depending on the type of the user (laboratory technician or the like), and therefore, a malicious user action cannot be prevented. On the other hand, uniformly and strictly imposing a viewing restriction on the user may reduce inspection efficiency.

In the invention, as a function of an automatic analyzer, a measure against information leakage for managing user viewing of specimen information based on reference classification information set for a patient specimen and reference authority set for a user without excessively reducing inspection efficiency is achieved.

### Solution to Problem

An automatic analyzer according to one embodiment of the invention includes: a display device configured to display specimen information; a storage device configured to store specimen ID data in which a specimen ID for identifying a specimen, an analysis item requested for the specimen ID, and reference classification information indicating presence or absence of a reference restriction for a measurement value of the analysis item of the specimen ID are recorded; and a computer configured to control display of specimen information on the display device.

The computer is configured to, when a logged-in user causes the display device to display specimen information on a predetermined specimen ID, refer to reference authority information set for each analysis item regarding an user ID of the user and reference classification information in the specimen ID data on the predetermined specimen ID, display a measurement value of an analysis item requested for the predetermined specimen ID on the display device when reference classification information on the analysis item is a classification indicating absence of a reference restriction in the specimen ID data, display a measurement value of an analysis item requested for the predetermined specimen ID on the display device when reference classification information on the analysis item is a classification indicating presence of a reference restriction in the specimen ID data and reference authority information set for the analysis item regarding the user ID is a classification indicating presence of a reference authority, and not display a measurement value of an analysis item requested for the predetermined specimen ID on the display device when reference classification information on the analysis item is a classification indicating presence of a reference restriction in the specimen ID data and reference authority information set for the analysis item regarding the user ID is a classification indicating absence of a reference authority.

### Advantageous Effects of Invention

Provided is an automatic analyzer having an information leakage prevention function for managing viewing of specimen information by a user. Other problems and novel features will become apparent from the description of the present specification and the accompanying drawings.

### Brief Description of Drawings

[FIG. 1] FIG. 1 is an overall configuration diagram of a multi-item chemical analysis device (automatic analyzer) and a system connected thereto.
[FIG. 2] FIG. 2 is a flowchart illustrating control of viewing of specimen information.
[FIG. 3A] FIG. 3A is an example of a reference authority setting screen for setting a reference authority for each user.
[FIG. 3B] FIG. 3B is an example of a reference authority setting screen for setting a reference authority for each user.
[FIG. 4] FIG. 4 is an example of a reference authority setting screen for setting a reference authority for each user classification.
[FIG. 5] FIG. 5 is an example of a user registration screen for setting a user classification for a user.
[FIG. 6] FIG. 6 is an example of an information disclosure setting screen.
[FIG. 7] FIG. 7 is an example of a data structure of patient data.
[FIG. 8] FIG. 8 is an example of a data structure of specimen ID data.
[FIG. 9] FIG. 9 is an example of a data structure of measurement result data.
[FIG. 10] FIG. 10 is a screen display example of measurement results when a restriction by a reference authority is absent.
[FIG. 11] FIG. 11 is a screen display example of measurement results when a restriction by a reference authority is present (non-display).
[FIG. 12] FIG. 12 is a diagram illustrating determination of whether specimen information can be referenced based on reference classification information and reference authority information.

### Description of Embodiments

An embodiment of the invention will be described below by taking a multi-item chemical analysis device that analyzes a plurality of request items of a specimen using a photometric method as an example. However, the invention is not limited to the multi-item chemical analysis device, and various automatic analyzers that handle patient specimens can be applied.

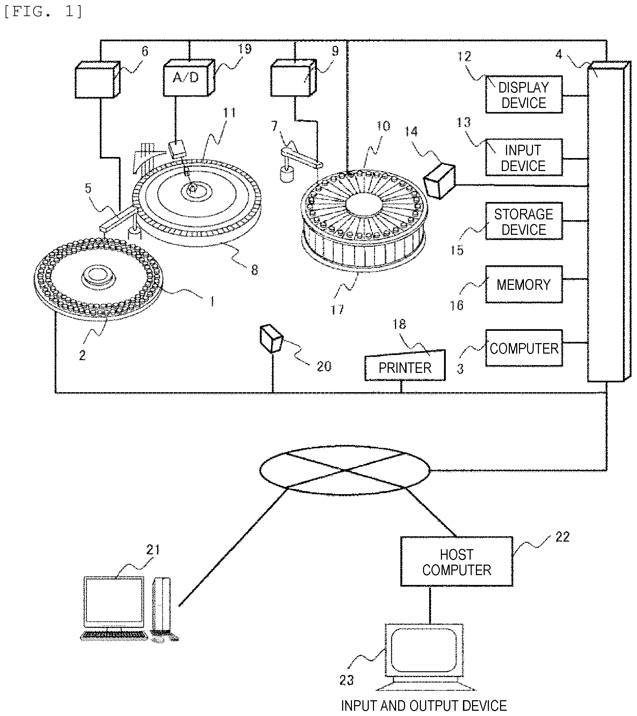

FIG. 1 is an overall configuration diagram of a multi-item chemical analysis device and a system connected thereto. A reaction disk 8 can be rotated intermittently, and a large number of reaction containers 11 made of a transparent material are arranged on the reaction disk 8 along a circumferential direction. Each of the reaction containers 11 is maintained at a predetermined temperature (for example, 37°C) by a constant-temperature bath. A large number of specimen containers 1 that accommodate biological samples such as blood and urine are placed on a sample disk 2 in a double manner along a circumferential direction in the illustrated example. Further, a sample dispensing mechanism 5 is disposed near the sample disk 2. This sample dispensing mechanism 5 includes a movable arm and a pipette nozzle attached to the movable arm. With this configuration, in the sample dispensing mechanism 5, when dispensing a sample, the pipette nozzle moves to a dispensing position to suck a predetermined amount of sample from the specimen container 1 located at a suction position on the sample disk 2, and discharges the sucked sample into the reaction container 11 located at a discharging position on the reaction disk 8. The operation of the sample dispensing mechanism 5 is performed while a liquid level detector 6 detects a liquid level of the specimen container 1 or the reaction container 11.

A plurality of reagent containers 10 to each of which a label showing reagent identification information, such as a barcode is adhered are placed on a reagent disk 17 along a circumferential direction of the reagent disk 17. The reagent container 10 accommodates a reagent liquid corresponding to an analysis item to be analyzed by the multi-item chemical analysis device. Further, a reagent ID reader 14 is attached to read the barcode shown on an outer wall of each reagent container 10 at the time of reagent registration. The read reagent information is registered in a memory 16 together with a position on the reagent disk 17.

A reagent dispensing mechanism 7 having substantially the same mechanism as the sample dispensing mechanism 5 is disposed near the reagent disk 17. When dispensing a reagent, a pipette nozzle in the reagent dispensing mechanism 7 aspirates a reagent liquid from the reagent container 10 corresponding to an inspection item of the reaction container 11 positioned at a reagent receiving position on the reaction disk 8, and discharges the reagent liquid to the corresponding reaction container 11. The operation of the reagent dispensing mechanism 7 is also performed while the liquid level is detected by a liquid level detector 9.

A light source is disposed near a center portion of the reaction disk 8 and a photometer is disposed on an outer circumferential side of the reaction disk 8 to form a photodetection system. A row of the reaction containers 11 rotates and moves to pass through a photometry position sandwiched between the light source and the photometer, and a reaction liquid of the sample and the reagent in each reaction container 11 is subjected to photometry every time the reaction disk 8 passes in front of the photometer during rotation. An analog signal of transmitted light or scattered light measured for each sample is converted into a digital signal by an A/D converter 19. An interior of the reaction container 11 for which the photometry is completed is cleaned by a reaction container cleaning mechanism disposed near the reaction disk 8, thereby making it possible to use the reaction container 11 repeatedly.

A computer 3 not only controls each mechanism of the multi-item chemical analysis device as described above, but also receives a detection signal from the photometer, which is converted into a digital signal by the A/D converter 19, to execute analysis of the sample according to the inspection item. Furthermore, a display device 12, an input device 13, a storage device 15, a printer 18, and a reader 20 are connected to the computer 3 via an interface 4. The reader 20 is used to read a specimen ID such as a barcode attached to the specimen container 1.

Furthermore, the multi-item chemical analysis device is connected to a host computer (clinical inspection information system) 22 provided in a consulting room or the like, and is capable of transmitting and receiving information via a network. The host computer 22 is also connected to an information management system 21 provided at a hospital reception desk or the like, and is capable of transmitting and receiving information via network communication. The information management system 21 and the host computer 22 each have a hardware configuration of a general information processing device. In other words, the information management system 21 and the host computer 22 each include a storage device such as an HDD (hard disk drive) or SSD (solid state drive), and execute information processing by loading program codes of software stored in the storage device into a main memory, and executing the loaded program codes by a processor.

In order to operate each piece of equipment using the display device 12 of the multi-item chemical analysis device or an input and output device 23 connected to the host computer 22, the user needs to log in. In this embodiment, by adding an authority to refer to patient specimen information to a user authority for operating a piece of equipment, the disclosure of the patient specimen information is kept to the minimum necessary, and reference by a user to the patient specimen information is controlled according to reference classification information given to each patient specimen.

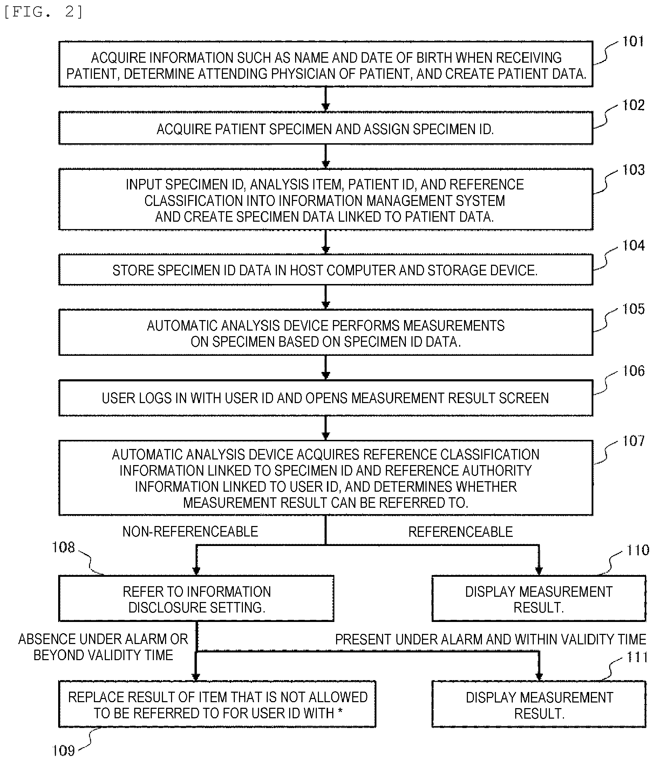

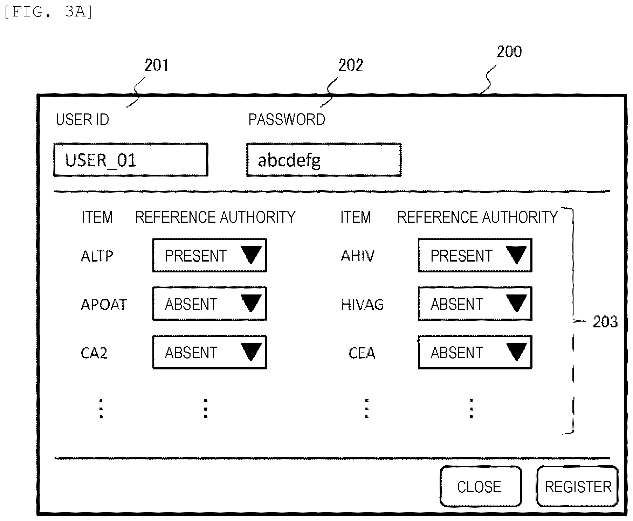

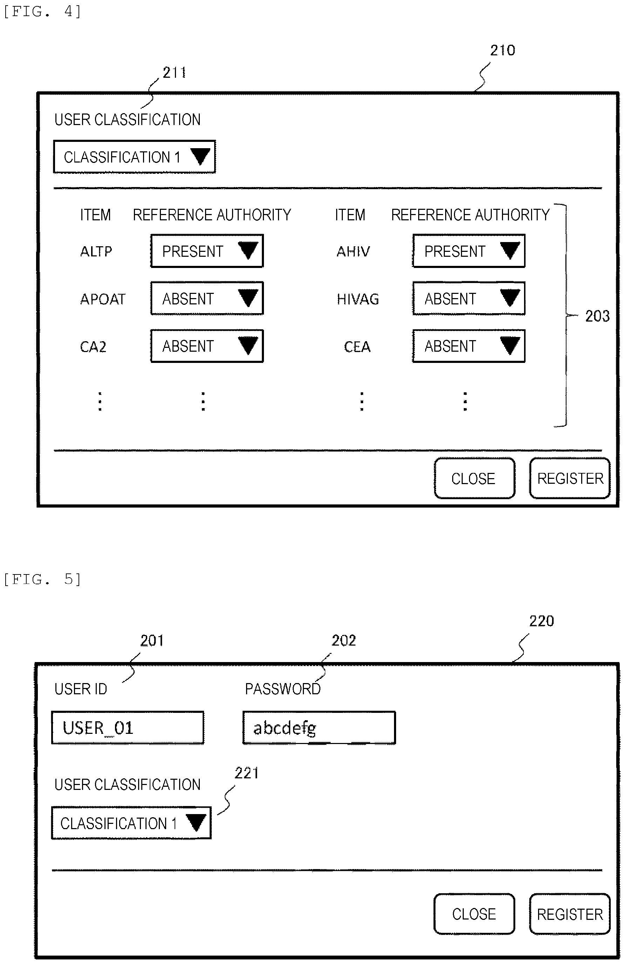

FIG. 2 shows a flowchart illustrating control of viewing of specimen information. A setting regarding a reference authority of a user (laboratory technicians or the like) who uses the automatic analyzer is performed in advance. This setting is performed through the host computer 22. FIG. 3A shows a reference authority setting screen. A user ID 201, a password 202, and reference authority information 203 are set for each user on the reference authority setting screen 200. Presence or absence of a reference authority is set for each analysis item. FIG. 4 shows an example of another reference authority setting screen. On a reference authority setting screen 210, each user classification 211 ("CLASSIFICATION 1" and "CLASSIFICATION 2") is registered at once. In this case, which user classification each user corresponds to is assigned using a user registration screen 220 illustrated in FIG. 5.

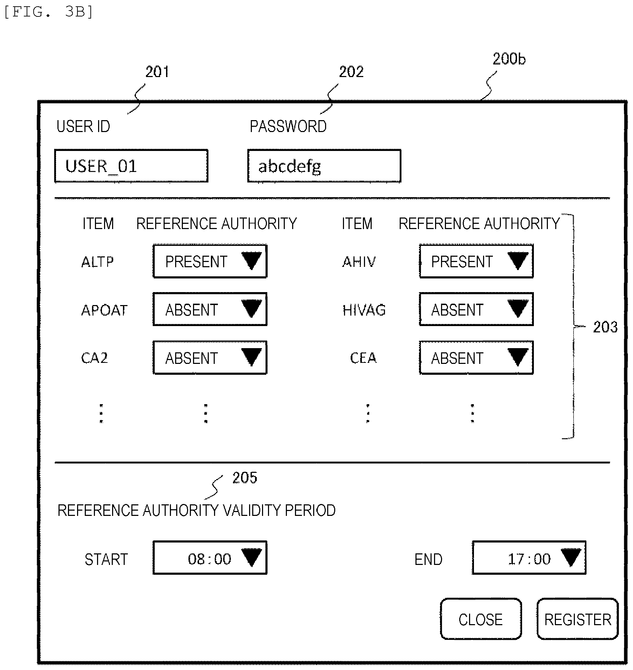

FIG. 3B shows an example of another reference authority setting screen. A reference authority validity period 205 can be set on a reference authority setting screen 200b. The reference authority validity period 205 is a time period during which the reference authority set for the user ID is valid. The reference authority for the user ID is uniformly treated as "ABSENT" beyond the validity period. In this case, it is sufficient to set a time period during which the set reference authority is valid and a time period during which the set reference authority is invalid, and therefore, an invalidity period may be set instead of setting the validity period. The reference authority for the user ID is uniformly treated as "ABSENT" during a time period specified as the invalidity period.

In the above example, an example in which the scope of the reference authority is specified for each analysis item is shown, but the scope of the reference authority may be set collectively for each inspection classification such as "biochemistry item" and "immunology item" which are classifications larger than an analysis item. In general, an analysis item that corresponds to an immunology item often includes information that directly relates to patient privacy. Therefore, it is also possible to avoid the complexity of setting in units of analysis item and to set the reference authority in large groups. The set reference authority information is stored in the storage device 15.

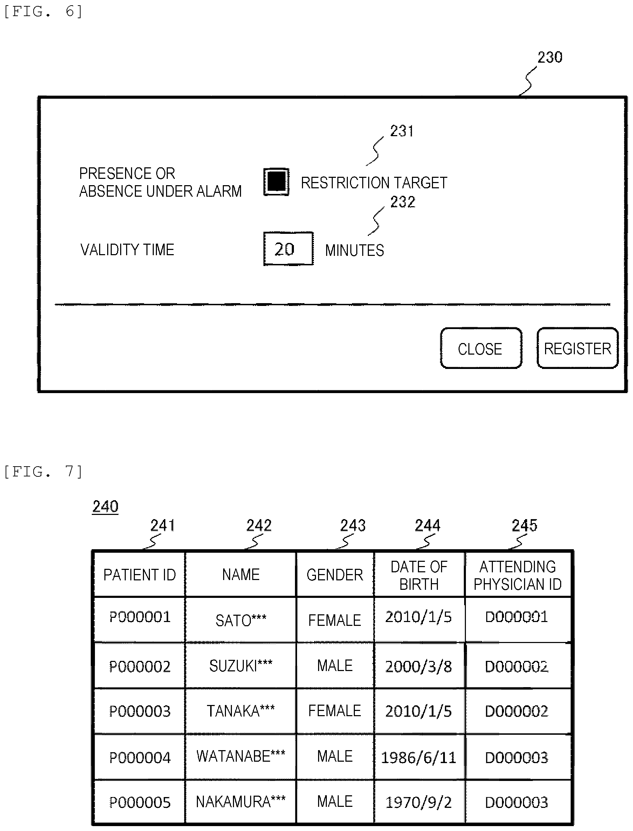

FIG. 6 shows an information disclosure setting screen 230. The information disclosure setting screen 230 is a screen for setting an exception of information disclosure based on the reference authority set as described above. A presence or absence under alarm 231 is used to set whether to grant the user an exception to the reference authority when the automatic analyzer determines that an item measurement result at the time of specimen measurement is an abnormal value. In a case where the alarm presence or absence under alarm 231 is specified, when an alarm indicating an abnormal value is output, even an analysis item for which the reference authority is set as absence is excluded from the restriction and the measurement value will be displayed. In a case where the presence or absence under alarm 231 is not specified, even though an alarm indicating an abnormal value is output, the measurement value of the analysis item for which the reference authority is set as absence is not displayed.

Even when the exception to the reference authority is granted, a restriction can be set to a display period, and a validity time 232 specifies a grace period until a disclosed measurement value is not displayed based on an original reference authority after a measurement result is output. For example, in the example shown in FIG. 6, the measurement value is displayed for 20 minutes after the measurement result is output, and during that period, even a user having no reference authority can take action to deal with the alarm. Accordingly, the alarm can be dealt with quickly. When the validity time 232 is not specified, the abnormal value is treated as not being restricted in display based on the reference authority.

The control of viewing of specimen information will be explained below with reference to the flowchart in FIG. 2. When receiving a new patient, an administrator determines an attending physician for the patient based on the patient, disease symptoms of the patient, or an inspection purpose, and registers these data in the information management system 21. FIG. 7 shows an example of a data structure of registered patient data 240. The patient data 240 includes a patient ID 241 (for example, a medical card number), personal information such as a name 242, a gender 243, and a date of birth 244 acquired from the patient or an insurance card of the patient, as well as a determined attending physician ID 245. The registered patient data 240 is transmitted from the storage device of the information management system 21 to the storage device of the host computer 22 and the storage device 15 of the automatic analyzer, and is stored therein (101).

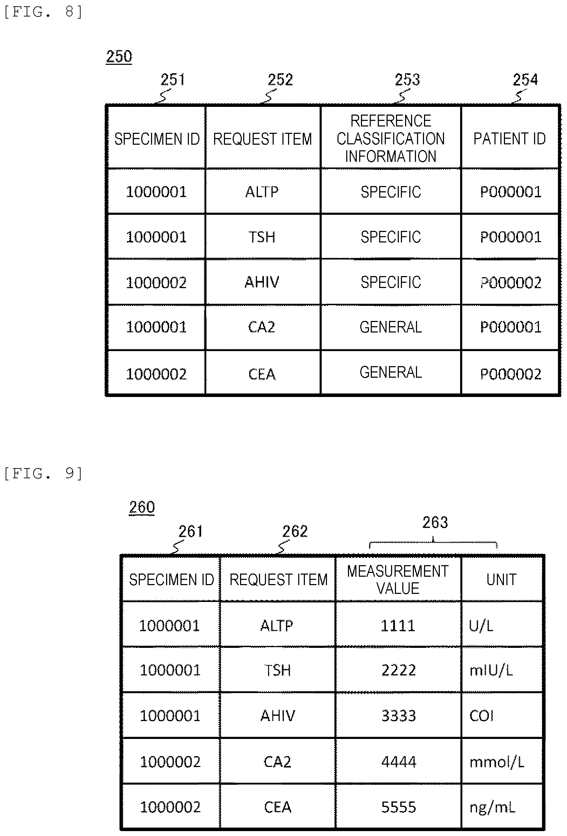

In order to uniquely identify a patient specimen, a specimen ID 251 is assigned to the patient specimen (102). The administrator registers the specimen in the information management system 21 and creates specimen data (103). FIG. 8 shows an example of a data structure of specimen ID data 250. The specimen ID data 250 includes a specimen ID 251, a request item 252 indicating an analysis item for which the automatic analyzer is requested to measure, reference classification information 253, and a patient ID 254. The specimen ID data 250 is linked to the patient data 240 using the patient ID 254 as a key, making it possible to acquire various information registered in the patient data 240.

In the reference classification information 253, one of two classifications is selected: "GENERAL" which does not impose a reference restriction regardless of the reference authority set for the user, and "SPECIFIC" which imposes a reference restriction in accordance with the reference authority set for the user.

The specimen ID data 250 stored in the storage device of the information management system 21 is stored in the storage device of the host computer 22 and the storage device 15 of the automatic analyzer (104).

The automatic analyzer performs measurements on the specimen based on the specimen ID data (105). When a measurement result is output, a measurement result 263 is stored in the storage device 15 for each specimen ID 261 and request item 262 as measurement result data 260 shown in FIG. 9. The measurement result is transmitted from the automatic analyzer to the host computer 22, and is also stored in the same format in the host computer 22.

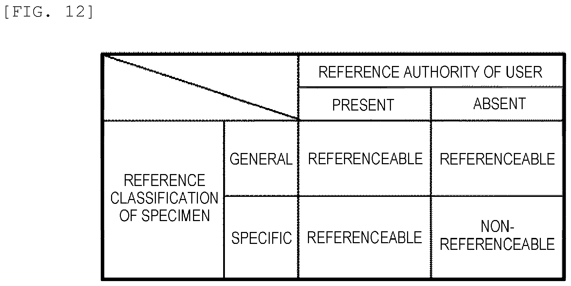

The user logs in the automatic analyzer with the user ID and opens a measurement result screen to confirm the measurement result (106). The automatic analyzer acquires the reference classification information 253 (see FIG. 8) linked to the specimen ID and the reference authority information 203 (see FIG. 3A) linked to the user ID, and determines whether each measurement result can be displayed on the measurement result screen (107). FIG. 12 shows a relationship between the reference classification of the patient specimen and the reference authority of the user. The patient specimen whose reference classification is "GENERAL" is displayed on the display device 12 of the automatic analyzer or the input and output device 23 of the host computer 22 regardless of the reference authority of the user, and the patient specimen whose reference classification is "SPECIFIC" is displayed on the display device 12 or the input and output device 23 only to a user who has the reference authority to this item.

As a result, the measurement result that is determined to be referenceable is displayed (110), and for the measurement result that is determined to be non-referenceable, information disclosure settings set on the information disclosure setting screen 230 (see FIG. 6) are referred to (108). Here, when an exception of information disclosure is set according to the contents on the screen in FIG. 6, the measurement result will be displayed when an alarm is output and the time is within the validity time (111), and the measurement result will not be displayed when no alarm is output or even though an alarm is output but the time is beyond the validity time (109). For example, the measurement result is replaced with a meaningless symbol string and displayed. From step 108 onward, actions are taken in accordance with the information disclosure settings.

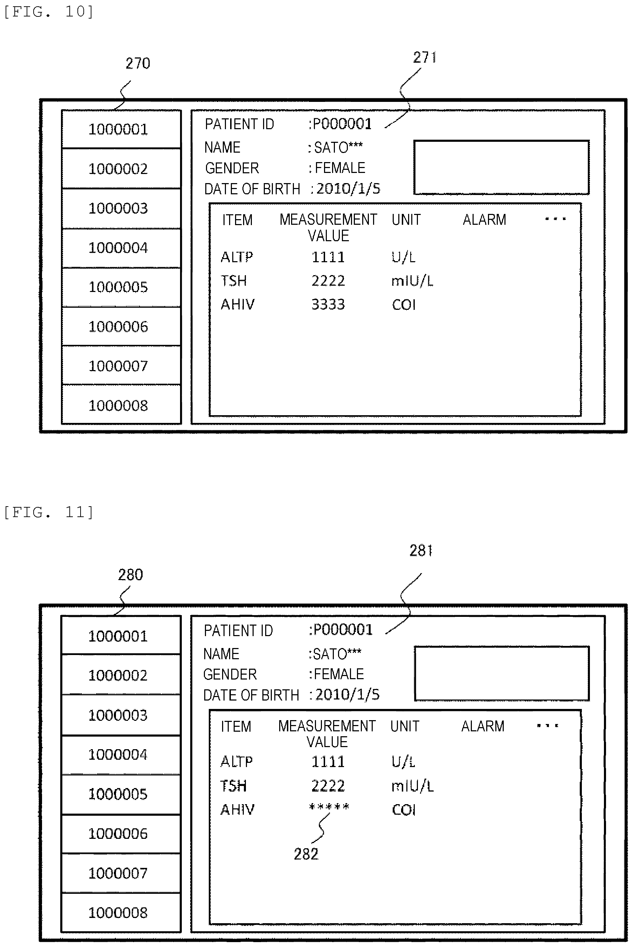

FIG. 10 shows a screen display example in which information on a patient specimen is displayed on the display device 12 of the automatic analyzer or the input and output device 23 of the host computer 22 for a user whose user ID has a reference authority for all analysis items. Personal information such as a patient ID, measurement results, a comment entry field, and the like are displayed in a specimen information display field 271 for a patient specimen selected from a specimen ID list 270 (for example, select a specimen ID "1000001" from the specimen ID list 270).

On the other hand, FIG. 11 shows a screen display example when information on a patient specimen are displayed to a user whose user ID does not have a reference authority for a specific item. By selecting a specimen ID from a specimen ID list 280, personal information such as a patient ID, measurement results, a comment entry field, and the like are displayed in a specimen information display field 281. At this time, a measurement value of the analysis items for which the user ID does not have a reference authority is masked so that it can be identified that reference is restricted due to an authority. In the example of FIG. 11, a mask 282 is set to a "*" string, but as long as the measurement value is not displayed, any other mark may be used. Alternatively, the measurement value may be filled in with a color without using the mark, or a character string such as "RESTRICTED" may also be displayed.

### Reference Signs List

1: specimen container
2: sample disk
3: computer
4: interface
5: sample dispensing mechanism
6, 9: liquid level detector
7: reagent dispensing mechanism
8: reaction disk
10: reagent container
11: reaction container
12: display device
13: input device
14: reagent ID reader
15: storage device
16: memory
17: reagent disk
18: printer
19: A/D converter
20: reader
21: information management system
22: host computer
23: input and output device
200, 200b, 210: reference authority setting screen
201: user ID
202: password
203: reference authority information
205: reference authority validity period
211, 221: user classification
220: user registration screen
230: information disclosure setting screen
231: presence or absence under alarm
232: validity time
240: patient data
241: patient ID
242: name
243: gender
244: date of birth
245: attending physician ID
250: specimen ID data
251: specimen ID
252: request item
253: reference classification information
254: patient ID
260: measurement result
261: specimen ID
262: request item
263: measurement result
270, 280: specimen ID list
271, 281: specimen information display field
282: mask

## Claims

1. An automatic analyzer comprising:
a display device configured to display specimen information;
a storage device configured to store specimen ID data in which a specimen ID for identifying a specimen, an analysis item requested for the specimen ID, and reference classification information indicating presence or absence of a reference restriction for a measurement value of the analysis item of the specimen ID are recorded; and
a computer configured to control display of specimen information on the display device, wherein
the computer is configured to
when a logged-in user causes the display device to display specimen information on a predetermined specimen ID, refer to reference authority information set for each analysis item regarding a user ID of the user and reference classification information in the specimen ID data on the predetermined specimen ID,
display a measurement value of an analysis item requested for the predetermined specimen ID on the display device when reference classification information on the analysis item is a classification indicating absence of a reference restriction in the specimen ID data,
display a measurement value of an analysis item requested for the predetermined specimen ID on the display device when reference classification information on the analysis item is a classification indicating presence of a reference restriction in the specimen ID data and reference authority information set for the analysis item regarding the user ID is a classification indicating presence of a reference authority, and
not display a measurement value of an analysis item requested for the predetermined specimen ID on the display device when reference classification information on the analysis item is a classification indicating presence of a reference restriction in the specimen ID data and reference authority information set for the analysis item regarding the user ID is a classification indicating absence of a reference authority.

2. The automatic analyzer according to claim 1, wherein
the reference authority information set for each analysis item regarding the user ID of the user is set collectively for each inspection classification.

3. The automatic analyzer according to claim 1, wherein
the reference authority information set for each analysis item regarding the user ID of the user is set for each time period.

4. The automatic analyzer according to claim 1, wherein an exception of information disclosure based on a reference authority when the measurement value of the analysis item requested for the predetermined specimen ID is an abnormal value and an alarm is output is set in the computer, and
when the alarm is output, the computer displays the measurement value of the analysis item on the display device even when the reference classification information on the analysis item requested for the predetermined specimen ID is a classification indicating presence of a reference restriction in the specimen ID data and the reference authority information set for the analysis item regarding the user ID is a classification indicating absence of a reference authority.

5. The automatic analyzer according to claim 4, wherein an expiration date is set for the exception of information disclosure, and
after the alarm is output and the expiration date is elapsed, the computer does not display the measurement value of the analysis item on the display device when the reference classification information on the analysis item requested for the predetermined specimen ID is a classification indicating presence of a reference restriction in the specimen ID data and the reference authority information set for the analysis item regarding the user ID is a classification indicating absence of a reference authority.

6. A specimen information display method in which a computer is used to control display of specimen information on a display device, the specimen information display method comprising:
storing, in a storage device, specimen ID data in which a specimen ID for identifying a specimen, an analysis item requested for the specimen ID, and reference classification information indicating presence or absence of a reference restriction for a measurement value of the analysis item of the specimen ID are recorded;
by the computer,
when a logged-in user causes the display device to display specimen information on a predetermined specimen ID, referring to reference authority information set for each analysis item regarding a user ID of the user and reference classification information in the specimen ID data on the predetermined specimen ID;
displaying a measurement value of an analysis item requested for the predetermined specimen ID on the display device when reference classification information on the analysis item is a classification indicating absence of a reference restriction in the specimen ID data;
displaying a measurement value of an analysis item requested for the predetermined specimen ID on the display device when reference classification information on the analysis item is a classification indicating presence of a reference restriction in the specimen ID data and reference authority information set for the analysis item regarding the user ID is a classification indicating presence of a reference authority; and
not displaying a measurement value of an analysis item requested for the predetermined specimen ID on the display device when reference classification information on the analysis item is a classification indicating presence of a reference restriction in the specimen ID data and reference authority information set for the analysis item regarding the user ID is a classification indicating absence of a reference authority.

7. The specimen information display method according to claim 6, wherein
the reference authority information set for each analysis item regarding the user ID of the user is set collectively for each inspection classification.

8. The specimen information display method according to claim 6, wherein
the reference authority information set for each analysis item regarding the user ID of the user is set for each time period.

9. The specimen information display method according to claim 6, wherein
an exception of information disclosure based on a reference authority when the measurement value of the analysis item requested for the predetermined specimen ID is an abnormal value and an alarm is output is set in the computer, and
when the alarm is output, the computer displays the measurement value of the analysis item on the display device even when the reference classification information on the analysis item requested for the predetermined specimen ID is a classification indicating presence of a reference restriction in the specimen ID data and the reference authority information set for the analysis item regarding the user ID is a classification indicating absence of a reference authority.

10. The specimen information display method according to claim 9, wherein
an expiration date is set for the exception of information disclosure, and
after the alarm is output and the expiration date is elapsed, the computer does not display the measurement value of the analysis item on the display device when the reference classification information on the analysis item requested for the predetermined specimen ID is a classification indicating presence of a reference restriction in the specimen ID data and the reference authority information set for the analysis item regarding the user ID is a classification indicating absence of a reference authority.
